# EUROPEAN PATENT APPLICATION

(11) **EP 1 170 012 A1**
(43) Date of publication of application: **09.01.2002**
(21) Application number: 99973693.7
(22) Date of filing: 03.12.1999
(51) Int. Cl.: A61K 31/704, A61K 45/00, A61P 25/00, A61P 43/00, G01N 33/15, G01N 33/50

(54) **CEREBROVASCULAR REGENERATION/RECONSTRUCTION PROMOTERS AND NERVE TISSUE SECONDARY DEGENERATION INHIBITORS COMPRISING GINSENOSIDE RB 1?**

(30) Priority: 19.02.1999 JP 4151799; 30.11.1999 JP 34085099
(71) Applicant: Japan Science and Technology Corporation, Kawaguchi-shi, Saitama 332-0012 (JP)
(72) Inventor: SAKANAKA, Masahiro, Onsen-gun, Ehime-ken 791-0204 (JP); TANAKA, Junya Suite 115 Ehime University, Onsen-gun Ehime-ken 791-0203 (JP); SATO, Kohji, Hamamatsu-shi Shizuoka-ken 433-8118 (JP)
(74) Representative: Cresswell, Thomas Anthony
(86) International application number: JP9906804
(87) International publication number: WO0048608

(57) **Abstract**

The present invention provides efficacious preparations comprising ginsenoside Rb₁ or its salt for intravenous administration, which are useful as vascular regeneration/reconstruction promoters and secondary nervous tissue degeneration inhibitors. These preparations are useful particularly for the regeneration and/or reconstruction of cerebrovascular networks after cerebral stroke and for the inhibition of secondary nervous tissue degeneration.

## Description

### Technical Field

The present invention relates to the pharmaceutical compositions comprising ginsenoside Rb₁, its metabolites or salts thereof, which are used for prevention, treatment or therapy of diseases caused by injuries to the nervous tissues or to the spinal cord. More particularly, the present invention pertains to the pharmaceutical compositions for prevention, treatment or therapy of the following disorders: diseases caused by secondary degeneration of the nervous tissues resulting from injuries to the nervous tissues, spinal cord injuries, head injuries, traumatic injuries to the nervous tissues or spinal cord, and demyelinating diseases caused by injuries to the nervous tissues or the spinal cord. Further, the present invention relates to the pharmaceutical compositions comprising ginsenoside Rb₁, its metabolites or salts thereof, which are used for promoting vascular regeneration and/or reconstruction, or relates to the pharmaceutical compositions comprising ginsenoside Rb₁, its metabolites or salts thereof, which are used for suppressing apoptosis or apoptosis-like cell death of oligodendrocytes. The present invention further relates to ginsenoside Rb₁ or its salts which are useful as promoters of vascular regeneration and/or reconstruction, or as inhibitors of secondary nervous tissue degeneration.

The present invention further relates to preparations for intravenous administration, which are used for prevention, treatment or therapy of the diseases or disorders described above. The present invention also relates to the use of ginsenoside Rb₁ or its metabolites as a leading compound(s) for exploring novel active components or compounds involved in prevention, treatment or therapy of diseases of the nervous tissues or the spinal cord, or for exploring novel brain cell-protective agents or novel neuroprotective agents.

### Background Art

Originally, methods for treatment of cerebral apoplexy (cerebral vascular diseases) are different among cerebral infarction (cerebral embolism and cerebral thrombosis), cerebral hemorrhage, transient ischemic attack and subarachnoidal hemorrhage. Strictly speaking, no effective countermeasures can be taken unless a cerebral CT inspection is performed. For example, thrombolytic agents can be used for the treatment of transient ischemic attack or cerebral infarction (cerebral embolism or cerebral thrombosis) but are contraindicated for the treatment of cerebral hemorrhage. However, cerebral apoplexy is a serious disease resulting in a permanent disorder of the brain's higher functional activities; and threatening the survival of patients if no treatment is performed for protecting nerve cells or neurons at risk in the lesion site as early as possible. Consequently, the treatment of cerebral apoplexy should be initiated without a moment's delay. Even the period of time during the CT inspection of the brain is, to put it strongly, a factor for reducing the possibility of recovery for patients with cerebral apoplexy. Surely, the treatment of acute cerebral apoplexy is a struggle not only against the cerebral apoplectic lesion but also against the time after its onset. Quite unfortunately, at present, whatever the disease type of cerebral apoplexy is (cerebral infarction, cerebral thrombosis, cerebral embolism, cerebral hemorrhage, subarachnoidal hemorrhage or transient ischemic attack), it is a fact that there are few known drugs showing potent effects, even if they are administered immediately after the onset of cerebral apoplexy.

The important thing in the treatment of cerebral apoplexy is not always related to the acute stages of the disease. Even if, as a result of administration of some potent neuroprotective agents, necrosis or apoptosis-like cell death of brain cells (including glial cells) or nerve cells can be temporarily prevented; and unless the regeneration and/or the reconstruction of the brain blood vessels in the lesion site can occur subsequently, the brain cells and the nerve cells (neurons) in the same site are likely to degenerate over a long time period. However, at present, almost no drugs for stimulating the regeneration and/or the reconstruction of the cerebral blood vessels are known. These facts can be explained more concretely by using the example of a cerebral infarct lesion caused by permanent occlusion of the cortical branch of the unilateral middle cerebral artery (MCA). When the MCA is permanently occluded, nerve cells or neurons in the site, to which nutrition is supplied only through the MCA (i.e. the nerve cells or neurons in the ischemic core) fall into necrosis and form a cerebral infarct lesion unless the MCA is soon recanalized and reperfused. Consequently, no drugs can rescue the brain tissue in the ischemic core. As previously described in the specifications of the JP98/365560 and PCT/JP/02550 (Brain cell or nerve cell-protective agents comprising ginsenoside Rb₁), and in the present specification, gradually progressing nerve cell death, which is different from necrosis, is defined as "apoptosis of nerve cells" or "apoptosis-like nerve cell death".

In the ischemic penumbra, since the supply of the blood flow from the MCA is terminated and the vascular networks in that lesion are extremely decreased; and since the blood supply continues, at least in part, from the cortical branches of the anterior cerebral arteries and the posterior cerebral arteries, the nerve cells or neurons in the ischemic penumbra may survive for a short period after permanent MCA occlusion in a critical condition. It is well known that, if no measures are taken, apoptosis-like nerve cell (neuron) death gradually develops in the ischemic penumbra, and the original lesion (ischemic penumbra) is totally and drastically changed to a cerebral infarct lesion. From the clinical standpoint, to rescue the nerve cells or neurons in the original ischemic penumbra is the most important thing to do. However, even if the nerve cells in that ischemic penumbra lesion can survive temporarily as a result of applying potent neuroprotective agents as described above, unless the vascular networks in that lesion, which are disrupted or decreased due to the permanent MCA occlusion, can be regenerated and/or reconstructed, the nerve cells in that lesion may eventually die. Consequently, with regard to the conditions required for neuroprotective agents, stimulation of the regeneration and/or the reconstruction of the disrupted vascular networks in the ischemic penumbra is essential in addition to a direct neuroprotective action.

A further problem in the treatment of cerebral apoplexy is the histological features or characteristics of the brain. Since each region in the brain constitutes complex information networks with the others through synaptic interactions, if one region is damaged, the damage frequently proceeds later in a staggered sequence to the other related regions connected synaptically with the original region (sometimes synaptic connection is designated as fiber connection). For example, it is reported that when a cerebral infarct lesion (the primary lesion) is developed in the unilateral cerebral hemisphere, nerve cell death (the secondary degeneration) is later induced in the thalamus of the same hemisphere, which keeps close synaptic connections with the cerebral infarct lesion. Subsequently, the patients deteriorate into cerebrovascular dementia depending on the progress of the unilateral thalamic atrophy or degeneration. Furthermore, if the function of the thalamus is damaged due to atrophy of the unilateral thalamus, tertiary degeneration is initiated in the other regions which have synaptic connections with the thalamus; then the cerebral functions of the patients with cerebral apoplexy may continue to deteriorate with the passage of time. In order to break the vicious circle resulting from such histological features or characteristics of the brain, drugs suppressing the above secondary neuronal degeneration are essential.

Ginsenoside Rb₁ is a compound having the following chemical structure (1): 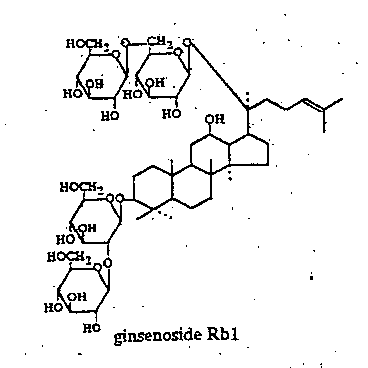 Ginsenoside Rb₁ is a known compound by a reference Shibata et al. (Shibata et al., Economic and medicinal plant research, World Scientific, Philadelphia, pp. 217-284, 1985).

Intraperitoneal administration of ginsenoside Rb₁ has been reported to show a tranquilizing action on the brain (Yoshimura H. et al., Eur. J. Pharmacol., 146, 291-297, 1988), but no mechanism of the action has been elucidated. In the central nervous system, the possibility has been raised that a mixture of ginsenoside Rb₁ and ginsenoside Rg₁ or ginsenoside Rb₁ or ginsenoside Rg₁ at the extracellular concentrations from 10⁻⁶ M to 10⁻⁷ M can be used for the treatment of Alzheimer's disease through activation of acetylcholine-containing neurons (US Patent No. 5,137,878: Composition and method for treatment of senile dementia). However, since it can not be said that the main cause of Alzheimer's disease is a functional disturbance of acetylcholine-containing nerve cells, this hypothesis has many problems to be solved. Moreover, the above US Patent does not address the question of whether ginsenoside Rb₁ can facilitate the survival of acetylcholine-containing nerve cells, namely the problem of whether ginsenoside Rb₁ can protect the acetylcholine-containing nerve cells or not.

The nerve cell-protective or neuroprotective action of ginsenoside Rb₁ has scarcely been elucidated until the studies on ginsenoside Rb₁ were initiated by the inventors of the present invention (Sakanaka and Tanaka). The inventors of the present invention (Sakanaka and Tanaka) have studied, until now, on the neuroprotective action of ginsenoside Rb₁ using the transient forebrain ischemia model of gerbils. It has been proved that in this forebrain ischemia model, occlusion of the bilateral common carotid arteries for 3 to 5 minutes while maintaining the brain temperature at 37°C results in a neuronal loss of the hippocampal CA1 pyramidal neurons (containing no acetylcholine) within one week after ischemia depending on the occlusion time (this event is called delayed neuronal death), and that the learning behavioral function of the ischemic animals is deteriorated (Wen T.-C. et al., Acta Neuropathol., 91, 15-22, 1996). These facts mean that the transient forebrain ischemia model of gerbils reflects the human pathologic condition of transient ischemic attack (TIA).

The one of the inventors of the present invention (Sakanaka) has proved that administering ginsenoside Rb₁ (10 mg/kg/day or 20 mg/kg/day: approximately 0.7mg/day or 1.4 mg/day calculated by estimating the body weight of a gerbil at about 70 g) into the peritoneal cavity of gerbils once a day for one week in advance can significantly prevent delayed neuronal death and learning disability caused by occlusion of the common carotid arteries for 5 minutes (Wen T.-C. et al., Acta Neuropathol., 91, 15-22, 1996). However, intraperitoneal administration of ginsenoside Rb₁ immediately after 3- or 5-minute occlusion of the common carotid arteries showed no effect (Wen T.-C. et al., Acta Neuropathol., 91, 15-22, 1996; Lim J.-H. et al., Neurosci. Res., 28, 191-200, 1997). Consequently, since transition rate and transportation rate to brain of peripherally (intraperitoneally) administered ginsenoside Rb₁ are thought to be very low, no clinical application of ginsenoside Rb₁ was kept in mind at that stage in view of the protection of hippocampal CA1 pyramidal neurons.

It has been reported by us (Sakanaka and Tanaka) that intracerebroventricular infusion of ginsenoside Rb₁ starting immediately after occlusion of the common carotid arteries for 3 or 3.5 minutes in place of the above peripheral (intraperitoneal) administration suppresses the delayed neuronal death and learning disability (Lim J.-H. et al., Neurosci. Res., 28, 191-200, 1997). Further, it has been proved by us (Sakanaka and Tanaka) that in spontaneous hypertensive stroke-prone (SH-SP) rats with permanent occlusion of the cortical branch of the unilateral middle cerebral artery (MCA) (cerebral infarction model of rats), intracerebroventricular infusion of ginsenoside Rb₁ immediately after permanent occlusion of the MCA caused a significant reduction of the infarcted area in the cerebral cortex and ameliorated the ischemia-induced place navigation disability of the animals (Zhang B. et al., J. Stroke Cerebrovasc. Dis., 7, 1-9, 1998).

Even though ginsenoside Rb₁ is effective in the direct intracerebroventricular infusion, however, it appears impossible to apply ginsenoside Rb₁ to human transient cerebral ischemic attack (TIA) and cerebral infarction due to the problems in the route of administration similarly to other peptide growth factors (Sakanaka M. et al., Proc. Natl. Acad. Sci. USA, 95, 4635-4640, 1998; Wen T.-C. et al., J. Exp. Med., 188, 635-649, 1998).

Concerning the mechanism of neuroprotective action by peripheral (intraperitoneal) administration of ginsenoside Rb₁, we (Sakanaka and Tanaka) have reported that a culture medium previously admixed with a low concentration (1 - 100 fg/ml) of ginsenoside Rb₁ reduces neuronal necrosis caused by a hydroxyl radical inducer (ferrous sulfate) (Lim J.-H. et al., Neurosci. Res., 28, 191-200, 1997; Zhang B. et al., J. Stroke Cerebrovasc. Dis., 7, 1-9, 1998). We have presumed from the first that ginsenoside Rb₁ decreases cell membrane lipid peroxides as a result of erasing hydroxyl radicals to protect cultured nerve cells, but on the basis of our recent studies (JP98/365560, PCT/JP99/02550: "Brain cell or nerve cell-protective agents comprising ginsenoside Rb₁"), this hypothesis has been found not always to be correct.

Several reports concerning the neuroprotective effect of ginsenoside Rb₁ have been made in culture experiments. For example, high concentrations (0.11-11 µg/ml) of ginsenoside Rb₁ reduce glutamate-mediated neurotoxicity to prevent neuronal cell death (Kim Y.-C., et al., J. Neurosci. Res., 53, 426-432, 1998), and a higher concentration, approximately 500 µM (550 µg/ml) of ginsenoside Rb₁ has a possibility to prevent apoptosis-like nerve cell death (Tanaka T. et al., The Ginseng Review, 24, 61-65, 1998; Takino I. et al., ibid., 25, 44-50, 1998). However, according to the results of our culture experiments (Sakanaka and Tanaka), high concentrations of ginsenoside Rb₁ have shown an increased neurotoxicity (Lim J.-H. et al., Neurosci. Res., 28, 191-200, 1997; Zhang B. et al., J. Stroke Cerebrovasc. Dis., 7, 1-9, 1998).

Furthermore, such high concentrations of ginsenoside Rb₁ can not be realized in an extracellular fluid in vivo, and we speculate that administration of large amounts of ginsenoside Rb₁ into human body to maintain the high extracellular concentrations of ginsenoside Rb₁ is impossible, considering its cost and adverse effects. Actually, from our experimental results (Sakanaka and Tanaka), it has been proven that a high dose of ginsenoside Rb₁ can not always provide preferable efficacy and effectiveness (Zhang B. et al., J. Stroke Cerebrovasc. Dis., 7, 1-9, 1998).

We have aimed at elucidation of the mechanism underlying the neuroprotective action of ginsenoside Rb₁ and at the invention relating to new efficacy and applicability of the said compound, and have demonstrated up to now the suppressive effect of low concentrations of ginsenoside Rb₁ on nerve cell death (JP98/365560, PCT/JP99/02550: "Brain cell or nerve cell-protective agents comprising ginsenoside Rb₁"). As the results, we have found that ginsenoside Rb₁, at such a markedly low concentration range never reported in the world as 1 fg/ml to 100 fg/ml, suppresses apoptosis-like nerve cell death by increasing the expression of a cell death-suppressing gene product Bcl-X_{L}.

Namely we have found that ginsenoside Rb₁ is the only one non-peptidic stimulator of Bcl-X_{L} expression in the world. Although ginsenoside Rb₁ at the concentration of 100 fg/ml showed a slightly suppressive action on the formation of lipid peroxides, no such effect was observed at a lower concentration range. Consequently, the hypothesis heretofore presented in relation to the action mechanism of ginsenoside Rb₁, namely the hypothesis that ginsenoside Rb₁ decreases cell membrane lipid peroxides as a result of erasing hydroxyl radicals to protect nerve cells, was found inappropriate.

Further, we have found that intravenous administration of ginsenoside Rb₁ exhibits unexpectedly a superior suppressive action against cerebral infarction and ameliorates infarction-induced place navigation disability (JP98/365560, PCT/JP99/02550: "Brain cell or nerve cell-protective agents comprising ginsenoside Rb₁").

However, if vascular regeneration and reconstruction do not occur in the ischemic brain tissue, i.e. the ischemic penumbra, which is relieved of entering cerebral infarction as a result of administering ginsenoside Rb₁, even after termination of the intravenous administration of ginsenoside Rb₁, there is a high possibility that new injuries to the brain may appear later in the same region after termination of the administration of ginsenoside Rb₁. In addition, even if the primary infarcted lesion of the cerebral cortex is ameliorated by the intravenous administration of ginsenoside Rb₁, unless the secondary degeneration in the thalamus which has close synaptic connections with the cerebral cortex, is inhibited, the effect and efficacy of intravenously administered ginsenoside Rb₁ may not be fully elicited.

We have found that the intravenous administration of ginsenoside Rb₁ promotes regeneration and/or reconstruction of the vascular networks in the ischemic penumbra and have completed the present invention. We have also found that the intravenous administration of ginsenoside Rb₁ suppresses the secondary degeneration of the thalamus which is generated after cerebrocortical infarction, and the secondary degeneration of the nervous tissues which occurs after spinal cord injury, and' have completed the present invention.

### Disclosure of the Invention

An object of the present invention is to provide pharmaceutical compositions or products promoting, in a superior manner, cerebrovascular regeneration and reconstruction by an intravenous administration after cerebral apoplexy as well as protecting the injured or damaged brain for a long period by inhibiting the secondary degeneration of the nervous tissues.

Another object of the present invention is to provide efficacious preparations for administration comprising ginsenoside Rb₁ or its salt useful as the promoters of cerebrovascular regeneration and/or reconstruction and as the inhibitors of secondary nervous tissue degeneration. More particularly, the present invention provides the pharmaceutical 'compositions comprising ginsenoside Rb₁ or its salt for promoting the cerebrovascular regeneration and/or reconstruction, or the pharmaceutical compositions comprising ginsenoside Rb₁ or its salt which inhibit the secondary nervous tissue degeneration. Further object of the present invention is to provide the preparations comprising ginsenoside Rb₁ or its salt for intravenous administration useful for long term therapy, prevention or treatment of cerebral or nervous diseases.

### Brief Description of Drawing

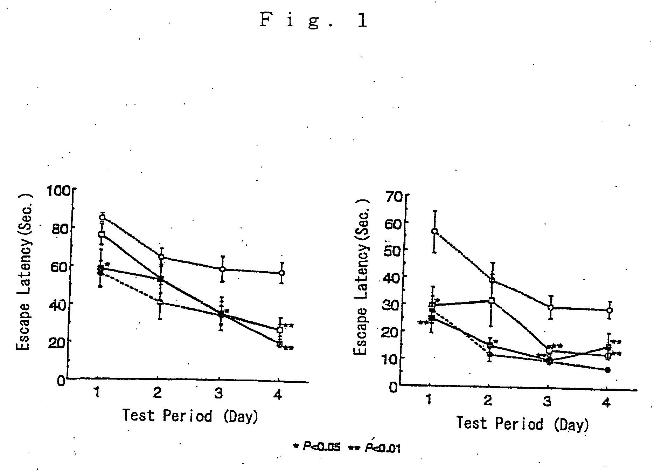

Fig. 1 shows the results of water maze tests using rats intravenously infused with ginsenoside Rb₁. The left drawing in Fig. 1 shows the results of water maze tests at the second week after MCA permanent occlusion and the right drawing shows the results of water maze tests at the fourth week after MCA occlusion. In Fig. 1, closed circles (●) indicate the results of rats with sham operation; and open circles (○) indicate the results of MCA-occluded rats infused with only physiological saline; closed squares (■) indicate the results of MCA-occluded rats infused with ginsenoside Rb₁ in a dose of 6µg/day and open squares (□) indicate the results of MCA-occluded rats infused with ginsenoside Rb₁ in a dose of 60µg/day. Data are shown by means ± SE. Statistical analysis is performed by ANOVA-Fisher's PLSD.

Fig. 2 is a figure showing ratios of cerebrocortical infarction in rats intravenously infused with ginsenoside Rb₁. Data are shown by means ± SE. Statistical analysis is performed by Mann-Whitney's U-test.

Fig. 3 consists of photographs instead of drawing, showing infarcted lesions in the cerebral cortex. A: MCA-occluded rat infused with physiological saline and B: MCA-occluded rat infused with ginsenoside Rb₁.

Fig. 4 is a schematic representation summarizing the results of examples 1, 2 and 3.

Fig. 5 is a drawing showing the region for measurement of blood vessel area in the non-infarcted ischemic penumbra of the parietal lobe in a 5 µm thick section of the brain..

Fig. 6 consists of differential interference contrast images instead of drawing, showing a photomicrograph from the penumbra on the ischemic side (i.e. ischemic penumbra) and a photomicrograph from the corresponding region on the intact side (control side).

Fig. 7 consists of photomicrographs instead of drawing, showing the thalamic VP nucleus. A: Sham-operated animal, B: ischemic animal administered with physiological saline; and C: ischemic animal administered with ginsenoside Rb₁ (60 µg/day). Bar indicates 100µm.

Fig. 8 consists of photographs instead of drawing, showing rats at 2 days after spinal cord (lower thoracic cord) injuries.

Fig. 9 indicates BBB score of rats administered with physiological saline and rats administered with ginsenoside Rb₁ (12 µg/day and 60 µg/day) at 7 days after spinal cord injuries.

### Best Mode for Carrying Out the Invention

The present invention relates to the pharmaceutical compositions comprising ginsenoside Rb₁, its metabolites or salts thereof for prevention, treatment or therapy of diseases caused by injuries to the nervous tissues or to the spinal cord. More particularly, the present invention pertains to the pharmaceutical compositions for prevention, treatment or therapy of the following disorders; diseases caused by secondary degeneration of the nervous tissues resulting from injuries to the nervous tissues, spinal cord injurie, diseases caused by traumatic injuries to the nervous tissues or the spinal cord, and demyelinating diseases caused by injuries to the nervous tissues or the spinal cord.

Ginsenoside Rb₁, its metabolites or salts thereof of the present invention have the actions to promote vascular regeneration and/or reconstruction, or to inhibit apoptosis or apoptosis-like cell death of oligodendrocytes. Consequently the present invention further relates to promoters of vascular regeneration and/or reconstruction, preferably the promoters of vascular regeneration and/or reconstruction after cerebral apoplexy, inhibitors of secondary nervous tissue degeneration, or to inhibitors of the apoptosis or apoptosis-like cell death of oligodendrocytes.

The present invention further relates to the pharmaceutical compositions comprising ginsenoside Rb₁, its metabolites or salts thereof for preventing or treating diseases caused by injuries to the nervous tissues or the spinal cord by promoting vascular regeneration and/or reconstruction, preferably by promoting cerebrovascular regeneration and/or reconstruction after cerebral apoplexy, or by inhibiting apoptosis or apoptosis-like cell death of oligodendrocytes.

Further, the present invention relates to the pharmaceutical compositions comprising ginsenoside Rb₁, its metabolites or salt thereof, for promoting vascular regeneration and/or reconstruction, the pharmaceutical compositions for prevention, treatment or therapy of the secondary degeneration of the nerve tissues, the pharmaceutical compositions for prevention, treatment or therapy of traumatic injuries to the nerve tissues or the spinal cord, the pharmaceutical compositions for inhibiting apoptosis or apoptosis-like cell death of oligodendrocytes, the pharmaceutical compositions for prevention, treatment or therapy of spinal cord injuries, and to the pharmaceutical compositions for prevention, treatment or therapy of demyelination.

The inventors of the present invention have found that ginsenoside Rb₁ or its metabolites are extremely effective for prevention, treatment or therapy of diseases of the nervous tissues or the spinal cord. Consequently, the present invention relates to the methods for exploring novel active compounds or components for prevention, treatment or therapy of diseases of the nervous tissues or the spinal cord by using ginsenoside Rb₁ or its metabolites as a leading compound(s).

The'present invention still further relates to the use of ginsenoside Rb₁ or its metabolites as the leading compound(s) for exploring novel active compounds or components for prevention, treatment or therapy of diseases of the nervous tissues or the spinal cord, and to the use of ginsenoside Rb₁ or its metabolites as the leading compound (s) for exploring novel brain cell-protective agents or novel neuroprotective agents. The present invention further relates to the compositions for prevention, treatment or therapy of diseases of the nervous tissues or the spinal cord as obtained by the methods or uses hereinbefore.

The present invention relates to the use of ginsenoside Rb₁, its metabolites or salt thereof for production of pharmaceutical compositions for prevention, treatment or therapy of diseases caused by injuries to the nervous tissues or to the spinal cord. The present invention further relates to the use of ginsenoside Rb₁, its metabolites or salt thereof for production of the pharmaceutical compositions for promoting vascular regeneration and/or reconstruction, the pharmaceutical compositions for prevention, treatment or therapy of the secondary degeneration of the nervous tissues, the pharmaceutical compositions for prevention, treatment or therapy of traumatic injuries to the nerve tissues or to the spinal cord, the pharmaceutical compositions for inhibiting apoptosis or apoptosis-like cell death of oligodendrocytes, the pharmaceutical compositions for prevention, treatment or therapy of spinal cord injuries, and to the pharmaceutical compositions for prevention, treatment or therapy of demyelination.

The pharmaceutical compositions of the present invention preferably contain ginsenoside Rb₁, its metabolites or salt thereof at low concentrations. The pharmaceutical compositions of the present invention is preferably in the form of parenteral administration such as intravenous administration or mucosal administration. More particularly, the pharmaceutical compositions of the present invention are preferably in the form of parenteral administration containing ginsenoside Rb₁, its metabolites or salt thereof at low concentrations.

The present invention further relates to preparations comprising ginsenoside Rb₁, its metabolites or salt thereof, preferably at low concentrations, for parenteral administration, preferably the preparations for intravenous administration, for prevention, treatment or therapy of the diseases described hereinbefore.

The pharmaceutical compositions of the present invention are preferably used as the preparations for intravenous administration, and any administration routes, for example, external preparations for topical use in lesion, topical injections for lesion, preparations for oral administration, nasal drops, eye drops, suppositories, subcutaneous injections, intracutaneous injections, intramuscular injections, inhalations, sublingual preparations and percutaneous absorption can be selected.

The present invention relates to preparations such as the preparations for intravenous administration and the external preparations for topical use in lesion for long term therapy, prevention or treatment of brain and nerve diseases the promoters of cerebrovascular regeneration and reconstruction or to the inhibitors of the secondary degeneration of the nervous tissues.

The inventors of the present invention have found for the first time that ginsenoside Rb₁ or its metabolites can promote vascular regeneration and/or reconstruction, especially facilitate cerebrovascular regeneration and/or reconstruction after cerebral apoplexy, or inhibit apoptosis or apoptosis-like cell death of oligodendrocytes. Consequently, the present invention raises the possibility that ginsenoside Rb₁ or its metabolites can be used as the leading compound(s) to explore other novel active compounds or to components for prevention, treatment or therapy of diseases caused by injuries to the nervous tissues or to the spinal cord. Further, any administration routes can be selected after making prodrugs by modifying a part(s) of the chemical structure of ginsenoside Rb₁. In addition, by the identification of target molecules of ginsenoside Rb₁ or its metabolites, novel compounds, which can modify functions of the target molecules, would be synthesized, leading to the development of drugs for treatment of spinal cord injuries, neurotrauma or traumatic injuries.

Consequently, the present invention provides ginsenoside Rb₁ or its metabolites as the leading compound(s) for exploring novel active compounds or components for prevention, treatment or therapy of the diseases described hereinbefore.

Ginsenoside Rb₁ of the present invention is a compound represented by the chemical structure depicted hereinbefore, and ginsenoside Rb₁ can be isolated and purified according to the method of Shibata et al. (Shibata S. et al., Economic and Medicinal Plant Research, World Scientific, Philadelphia, pp. 217-284, 1985). Ginsenoside Rb₁ purified by such a method has a purity more than 98%, which has been confirmed by thin-layered chromatography and nuclear magnetic resonance spectrum (Kawashima Y. and Samukawa K., J. Med. Pharmacol. Soc. Wakan-Yaku, 3, 235-236, 1986).

Ginsenoside Rb₁ of the present invention can be used in its free form, but can be used as its suitable salts. Its solvates such as hydrates can also be used.

The concentrations of ginsenoside Rb₁ used in the present invention are preferably low, as described in JP98/365560 and PCT/JP99/02550 (Brain cell or nerve cell-protective agents comprising ginsenoside Rb₁), more concretely, its concentration in extracellular fluid in lesion is 1 ng/ml or less, preferably 1 pg/ml or less, or more preferably 100 fg/ml or less. The preparations for intravenous administration of ginsenoside Rb₁ of the present invention should preferably be adjusted so that the concentrations of ginsenoside Rb₁ in the extracellular fluid of the lesion tissue of patients are maintained in the concentration range hereinabove described. Sufficient favorable effects of the pharmaceutical compositions and preparations of the present invention can be obtained even at the concentration of 1 - 100 fg/ml in the extracellular fluid of the lesion tissue.

It was already found that intravenously administered ginsenoside Rb₁, unlike peripherally (intraperitoneally) administered ginsenoside Rb₁, was transferred rapidly to the central nervous system (JP98/365560 and PCT/JP99/02550: Brain cell or nerve cell-protective agents comprising ginsenoside Rb₁). The preparations for intravenous administration of the present invention may optionally be the preparations, which can be directly administered intravascularly, preferably intravenously. The preparations can be used for single intravenous infusion or continuous intravenous infusion after dissolving the present 'pharmaceutical compositions with physiological saline, distilled water, phosphate buffer, glucose solution, liposome or lipid microsphere. The preparations can also be a formulation, which can be used by adding to preparations for intravenous administration such as a composition for drip infusion. Further, a part(s) of the chemical structure of ginsenoside Rb₁ is modified to prepare prodrugs and a suitable route of administration or a suitable method for administration can be selected. For example, a hydroxyl group(s) in ginsenoside Rb₁ is esterified to prepare the prodrug(s), and the resulting prodrug(s) is likely to pass through the blood brain barrier, subsequently is hydrolyzed by endogenous esterase, thereby to increase the amount of intracerebrally transferred ginsenoside Rb₁.

As described in JP98/365560 and PCT/JP99/02550 ("Brain cell or nerve cell-protective agents comprising ginsenoside Rb₁"), intravenous administration of ginsenoside Rb₁ can reduce the infarcted area to about 1/4 in comparison with that of a non-administered control group, as well as having a unique action mechanism that is to enhance the expression of a cell death-suppressing factor Bcl-X_{L}. It also protects nerve cells or neurons in the brain. Consequently, it can be applied as neuroprotective agents for not only acute and chronic cerebral infarction (cerebral thrombosis or cerebral embolism) but also acute phase or chronic phase of cerebral hemorrhage and subarachnoidal hemorrhage or transient cerebral ischemic attack. Namely, ginsenoside Rb₁, which does not promote bleeding tendency, is the drug or pharmaceutical composition, which can be administered intravenously by drip infusion in an ambulance car to a patient suspected to suffer from cerebral apoplexy. Administration of ginsenoside Rb₁ to patients with cerebral infarction before thrombolytic therapy would ameliorate prognosis of the patients.

In addition, ginsenoside Rb₁ of the present invention can not only reduce the infarcted area to about 1/4 as a result of intravenous administration for a maximum of 28 days, but can also cause recovery of the damaged and reduced vascular networks in the ischemic penumbra to almost a normal state. 'Consequently, intravenous administration of ginsenoside Rb₁ stimulates the regeneration and/or reconstruction of the damaged and/or reduced cerebrovascular networks after cerebral apoplexy, and once rescued by intavenous administration of ginsenoside Rb₁ the brain tissues in the ischemic penumbra can function in a normal manner presumably for good even after the termination of intravenous administration of the pharmaceutical composition or drug. Ginsenoside Rb₁ of the present invention is expected to protect the injured or damaged brain through an indirect and long term protective mechanism that is to facilitate the regeneration and reconstruction of cerebrovascular networks, and also provides a direct protective effect on nerve cells through enhancement of the expression of Bcl-X_{L} protein and suppression of apoptosis-like nerve cell death. As such, ginsenoside Rb₁ appears to be the first compound, in the world which can reduce the infarcted area to about 1/4 by intravenous infusion, not only during the acute phase of cerebral infarction, but also at one month after the onset of cerebral infarction. Consequently, in the future, various protective agents for brain cells or nerve cells may be newly developed by using ginsenoside Rb₁ or its metabolites as a leading compound(s).

In the general clinical field, there are many cases, in which the higher nervous function is continuously deteriorated even though no new ischemic brain attack is noted after the first onset of cerebral infarction. Especially there are cases with continuously deteriorating sequelae after cerebral apoplexy. A reason for this may be that the regeneration or reconstruction of the damaged or reduced cerebrovascular networks caused by cerebral apoplectic attack (brain attack) is sometimes insufficient. Intravenous administration or nasal administration of ginsenoside Rb₁ is expected to exhibit conspicuous effects in ameliorating such sequelae after cerebral apoplexy.

Since the intravenous administration of ginsenoside Rb₁ of the present invention exhibits a novel effect and efficacy in promoting vascular regeneration and/or reconstruction, it may also be effective for treatment of other diseases with symptoms of blood flow disorders (e.g. aortitis syndrome, acute peripheral arterial embolism, thromboangitis obliterans, arteriosclerosis obliterans, Raynaud's disease or Raynaud's syndrome) . The efficacy of ginsenoside Rb₁ to inhibit cell death in tissues suffering from blood flow disorders and in diseases with major symptoms of these blood flow disorders should be kept in mind. Consequently, ginsenoside Rb₁ is expected to reduce tissue injuries resulting from blood flow disorders of the ' peripheral tissues through at least two action mechanisms.

'Since the pharmaceutical composition comprising ginsenoside Rb₁ inhibits the development of secondary lesions in the brain regions which have synaptic conjunctions with the primary brain lesion, it also exhibits efficacy for the prevention of the secondary lesions of many neurodegenerative diseases (e.g. Alzheimer's disease, Pick's disease, spinocerebellar degeneration, Parkinson's disease, chorea, polyglutamine diseases, amyotrophic lateral sclerosis or multiple sclerosis), and is expected to improve QOL (Quality of Life) for patients as a result of reducing progression of higher nervous function disorders caused by these diseases. As described in JP98/365560 and PCT/JP99/02550 ("Brain cell or nerve cell-protective agents comprising ginsenoside Rb₁"), the pharmaceutical composition comprising ginsenoside Rb₁ is thought to exhibit efficacy against the primary lesions of these neurodegenerative diseases through its inhibitory effect on apoptosis-like nerve cell death and through upregulation of' Bcl-X_{L} expression.

Further, the intravenous administration of ginsenoside Rb₁ of the present invention markedly ameliorates paralysis of animals with spinal cord injuries. It is well known that the nervous tissues are more vulnerable to trauma than any other peripheral tissues. The fact that the pharmaceutical composition comprising ginsenoside Rb₁ exhibits conspicuously favorable effects for therapy and treatment of spinal cord injuries, indicates that ginsenoside Rb₁ is effective for the treatment of traumatic injuries to the peripheral tissues as well as the central nervous tissues. As shown in the following example 4, in rats with spinal cord injuries in which compression was loaded to the lower thoracic spinal cord, intravenous administration of ginsenoside Rb₁ ameliorated paralysis of both lower limbs (paraplegia) and enabled the rats to stand up after spinal cord injuries. The rats with spinal cord injuries, to which only physiological saline (i.e. vehicle) was administered, remained paralyzed in both lower limbs and could not stand up. In addition, intravenous administration of Solu-Medol (methylprednisolone), which is used for treatment of spinal cord injuries at present, could not ameliorate paralysis of both lower limbs (paraplegia) in rats with spinal cord injuries. On the basis of these findings, the therapeutic effect of ginsenoside Rb₁ on spinal cord injuries is thought to be the most potent among the compounds so far examined. Consequently, it is expected that, in the future, ginsenoside Rb₁ or its metabolites will be used as a leading compound(s) in the development of various remedies for spinal cord injuries, neurotrauma and traumatic injuries.

Further, a specific feature of the pharmaceutical preparation or composition comprising ginsenoside Rb₁ of the present invention, which should not be overlooked, is the fact that it does not show any adverse effects. For example, as described in JP98/365560 and PCT/JP99/02550 ("Brain cell or nerve cell-protective agents comprising ginsenoside Rb₁"), even though ginsenoside Rb₁ is added to normal cultured nerve cells or neurons, which are not treated with sodium nitroprusside (SNP), a nitric oxide monoxide donor, it shows no effect on normal metabolic activity. Moreover, ginsenoside Rb₁ at low. extracellular concentrations (1 - 100 fg/ml) protects only nerve cells against SNP-induced injuries. Consequently, ginsenoside Rb₁ does not affect normal neuronal functions but can give a favorable effect only on lesion tissue. This point can be emphasized as a superior property of ginsenoside Rb₁ than glutamate receptor antagonists under developing as neuroprotective agents at present.

It has also been reported that no effects of intracerebroventricular administration of ginsenoside Rb₁ on brain temperature, cerebral blood flow and blood pressure are observed (Lim J.-H. et al. Neurosci. Res., 28, 191-200, 1997; Zhang B. et al., J. Stroke Cerebrovasc. Dis., 7, 1-9, 1998). We have confirmed that intravenous infusion of ginsenoside Rb₁ in rats, in a dose of 60 µg/day, does not affect the cerebral blood flow. It is also known that ginsenoside Rb₁ does not promote bleeding tendency. No adverse effects were detected within a range of careful observation on animals, to which ginsenoside Rb₁ of the present invention was administered.

As described in JP98/365560 and PCT/JP99/02550 ("Brain cell or nerve cell-protective agents comprising ginsenoside Rb₁"), ginsenoside Rb₁ of the present' invention, when administered to rats with permanent MCA occlusion (body weight about 300 g) in the doses of 6 µg and 60µg/day, caused reduction of the cerebral infarction area and ameliorated ischemia-induced place navigation disability (cerebrovascular dementia). Furthermore, ginsenoside Rb₁ of the present invention, in its equivalent doses, promotes cerebrovascular regeneration and/or reconstruction in the non-infarcted ischemic penumbra of the rats with permanent MCA occlusion, and significantly inhibits the secondary lesion of the thalamus (secondary thalamic degeneration) in addition to reducing cerebrocortical infarct lesion (the primary ischemic lesion). Intravenous administration of ginsenoside Rb₁, in the dose of 60 µg/day or 12 µg/day, to rats with spinal cord (lower thoracic spinal cord) injuries significantly ameliorates paralysis or paraplegia.

Based on these experimental results, the dose range of ginsenoside Rb₁ to human patients with cerebral apoplexy (body weight 60 kg) is calculated as 1.2 mg - 12 mg/day. Consequently, daily doses of the pharmaceutical composition of the present invention to human patients with cerebral apoplexy or spinal cord injuries is, although depending on individual differences and on the severity of diseases among patients, 0.1 mg or more, preferably 1 mg or more, more preferably 10 mg or more. However, since required dose amount per body weight is, generally, decreased as the body weight of animals increases, a dose of 1/10 or less of this amount is thought to exhibit sufficient effects on human. When ginsenoside Rb₁ is used for prevention, therapy or treatment of diseases other than central nervous system (CNS) diseases, it is preferable to select the equivalent dose of the above, or doses of 1/10 to 1/100,000 thereof. Since the pharmaceutical composition of the present invention has less adverse effect, it can be administered considerably in large amount as an upper limit of dosage, and the upper limit of dosage is 1 g or less/day, preferably 0.1 g or less/day.

The method for administration of the pharmaceutical compositions of the present invention is intravascular administration, preferably intravenous administration and the amount of administration described above can be administered consecutively or repetitively. Ginsenoside Rb₁, an active composition or component of the present invention is a sort of saponin, and can be formulated by the conventional methods. For example, the aqueous pharmaceutical composition of the present invention can be prepared as a preparation for intravenous administration by dissolving lyophilized ginsenoside Rb₁ crystals or powders in physiological saline, distilled water, phosphate buffer or glucose solution. Lipid microspheres or liposome preparation can also be used. The concentrations of the pharmaceutical compositions comprising ginsenoside Rb₁ or its salt in the preparations for intravenous administration can optionally be adjusted unless so high, for example 0.01-10 mg/ml, preferably 0.1-1 mg/ml.

In the animal experiments of the present invention, ginsenoside Rb₁ was intravenously administered continuously for 28 days after permanent occlusion of the cortical branch of the left middle cerebral artery (MCA). In the actual case of acute cerebral apoplexy, unless any treatment is given after the onset of cerebral apoplexy, rapid progress in cerebrovascular damage, slough and degeneration in the ischemic penumbra is noted within 2 weeks. As a result, the infarct lesion is expanded because the primary lesion as well as the secondary lesion (ischemic penumbra) becomes irreversible. Consequently, if ginsenoside Rb₁ is administered during at least this period, it can be useful for the regeneration and reconstruction of vascular networks in the damaged ischemic penumbra and for suppression of the secondary lesion.

The present invention takes the initiative in addressing the regeneration and/or reconstruction of damaged and degenerated cerebral blood vessels by intravenous administration of ginsenoside Rb₁. The fact that ginsenoside Rb₁ promotes cerebrovascular regeneration and reconstruction indicates that ginsenoside Rb₁ is effective in promoting the regeneration and reconstruction of not only blood vessels of the nervous tissues but also those of the peripheral tissues. Specifically, ginsenoside Rb₁ is expected to exhibit efficacy against myocardial infarction, angina pectoris, aortitis syndrome, acute peripheral artery occlusion, thromboangitis obliterans, arteriosclerosis obliterans, Raynaud's disease and Raynaud's syndrome. This is explained in more detail by using the example of myocardial infarction. If a branch of the coronary artery is permanently occluded and is not reperfused, the myocardial cells, to which nutrition is supplied only by the permanently occluded coronary artery branch, enter necrosis.

However, myocardial cells (myocytes) near the necrotic (infarcted) area, such as cells to which blood is supplied, in part, from the other coronary artery branches, can survive as a result of intravenously infused ginsenoside Rb₁-mediated upregulation of the cell death (apoptosis)-suppressing gene (Bcl-X_{L}) expression (JP98/365560 and PCT/JP99/02550: "Brain cell 'or nerve cell-protective agents comprising ginsenoside Rb₁"). Subsequently, as a result of ginsenoside Rb₁-induced vascular regeneration and reconstruction at the same site, the myocytes can be permanently rescued from cell death. Ginsenoside Rb₁ protects patients who suffered from myocardial infarction, but unfortunately underwent no coronary artery bypass or PTCA (percutaneous transluminal coronary angioplasty). It does this through at least two different actions as described above, and is expected to contribute to a reduction in the infarcted lesion. Needless to say, if ginsenoside Rb₁ is administered intravenously to patients, who have developed myocardial infarction before they undergo coronary artery bypass or PTCA, the prognosis of the patients is significantly improved. Furthermore, for patients with diseases of peripheral tissues, the effects and efficacy of ginsenoside Rb₁ may be elicited at doses equivalent to doses used for brain diseases, or even at 1/10 to 1/100,000 of those doses.

In this regard, matters which should not be forgotten are that in many cases of patients with myocardial infarction, the pumping function of the heart has deteriorated to cause insufficient cerebral blood flow. This sometimes leads to irreversible brain damage. The intravenous administration of ginsenoside Rb₁ contributes to improvement in QOL (Quality of Life) for patients with myocardial infarction, since it protects brain cells or nerve cells against cerebral blood flow failure as described in JP98/365560 and PCT/JP99/02550 ("Brain cell or nerve cell-protective agents comprising ginsenoside Rb₁").

Next, the actions of intravenously administered ginsenoside Rb₁ are explained in detail.

First, we examined the actions of intravenous infusion of ginsenoside Rb₁. For this purpose, for example, male SH-SP rats, weighing 250 - 300 g at the age of 12-13 weeks were used. The animals were bred in an air-conditioned room with a 12:12 hour light-dark cycle, and water and feeds were supplied ad libitum. The cortical branch of the left middle cerebral artery (MCA) was coagulated and cut under inhalation anesthesia. A single intravenous administration of ginsenoside Rb₁ dissolved in physiological saline was conducted immediately after MCA permanent occlusion (6µg or 60µg), thereafter continuous intravenous administration of ginsenoside Rb₁ was performed for 28 days by using Alza osmotic mini-pump (6µg/day or 60µg/day).

Control animals with MCA occlusion (ischemic control animals) and sham-operated animals were administered with the same amount of physiological saline.

After MCA permanent occlusion, according to the method of the inventors of the present invention (Sakanaka and Tanaka) (Igase, K. et al., J. Cereb. Blood Flow Metab., 19, 298-306, 1999; Zhang B. et al., J. Stroke Cerebrovasc. Dis., 7, 1-9, 1998), water maze tests were performed for 4 days at the 2nd week and the 4th week, respectively, and the place navigation abilities of SH-SP rats were determined.

Results are shown in Fig. 1. The left drawing in Fig. 1 shows the results of the 2nd week and the right drawing shows the results of the 4th week after permanent MCA occlusion. In Fig. 1, closed circles (●) indicate the results of rats with sham operation; and open circles (○) indicate the results of MCA-occluded rats administered with only physiological saline; closed squares (■) indicate the results of MCA-occluded rats administered with ginsenoside Rb₁ in a dose of 6µg/day and open squares (□) indicate the results of MCA-occluded rats administered with ginsenoside Rb₁ in a dose of 60*µ*g/day.

As shown in Fig. 1, the place navigation disability after MCA permanent occlusion (after cerebral infarction) was significantly improved by ginsenoside Rb₁ infusion in groups of cerebral infarction administered with ginsenoside Rb₁ as compared with a group of cerebral infarction administered with physiological saline. Especially, in the water maze tests at the 2nd week and at the 4th week after MCA occlusion, the low dose of ginsenoside Rb₁ significantly ameliorated the learning disability on the 3rd day and on the 4th day, and the high dose of ginsenoside Rb₁ on the 4th day at the 2nd week and on the 3rd and 4th days at the 4th week after MCA occlusion: Significant effects were also noted on the 1st day at the 4th week in the high dose and the low dose groups, respectively. No significant differences in swimming speed of SH-SP rats were detected among the four experimental groups.

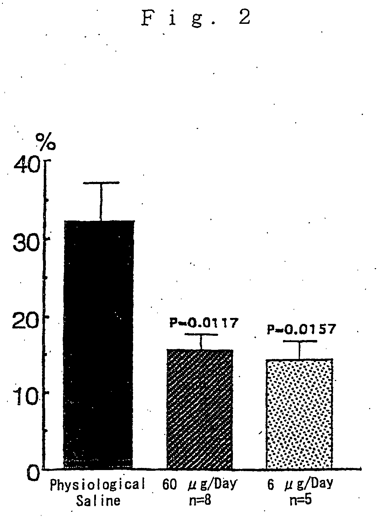

After the water maze tests at the 4th week, the SH-SP rats were anesthetized with chloral hydrate, and they were perfused and fixed transcardially with 0.1 mole phosphate buffer containing 4% paraformaldehyde. The brains were dissected out and cerebrocortical infarcted areas were photographed. Areas of the left cerebral hemispheres and the left cerebrocortical infarct lesions were measured on the photographs by using an image analysis device. The left cerebrocortical infarcted areas were divided by the left cerebral hemispheric areas to calculate ratios of the cerebrocortical infarction (%). Results are shown in Fig. 2.

As shown in Fig. 2, the ratio of cerebrocortical infarction was significantly reduced in the groups of cerebral infarction with intravenous administration of ginsenoside Rb₁ as compared with the group of cerebral infarction with administration of physiological saline. Since the ratio of cerebrocortical infarction is calculated based on the area of infarction, and the mean value of the ratio in the groups intravenously administered with ginsenoside Rb₁ is reduced to about 50% or less compared with that of the group administered with physiological saline, actual volume of infarction appears to be reduced to about 1/4 by intravenous administration of ginsenoside Rb₁.

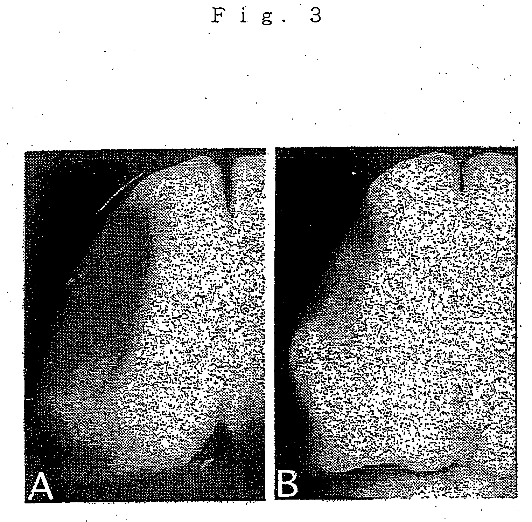

An actual case of cerebral infarct area of the group administered with physiological saline and an actual case of cerebral infarct area of the group administered with ginsenoside Rb₁ (6µg/day) are shown in Fig. 3A and Fig. 3B, respectively.

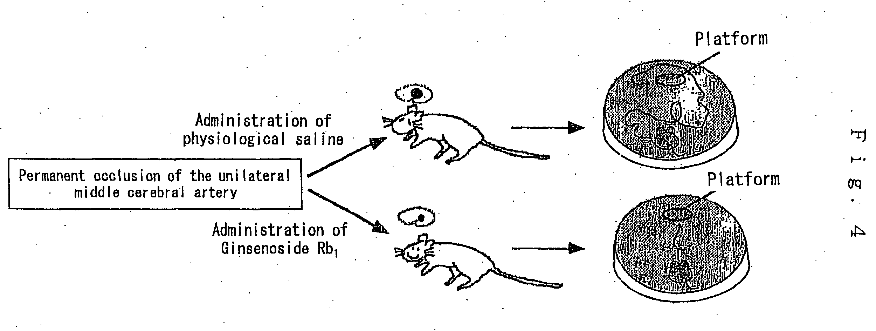

Fig. 4 is a schematic drawing summarizing the results of the present experiments. In rats administered with physiological saline, the size of cerebral infarction remained large and it took a long time for the rats to escape onto the goal platform in the water maze tests. Contrary, in rats administered with ginsenoside Rb₁ of the present invention, the infarct area was reduced, and as a result, in the water maze tests, only a short time was required for the rats to arrive at the goal platform.

According to the paper in the past by the inventor (Sakanaka) using the transient forebrain ischemia model of gerbils (Wen T.-C., et al., Acta Neuropathol., 91, 15-22, 1996), even if intraperitoneal administration of ginsenoside Rb₁ (10 mg/kg/day or 20 mg/kg/day) was performed before ischemic loading, only about 30% of hippocampal CA1 pyramidal neurons could be rescued. In addition, intraperitoneal administration of ginsenoside Rb₁ in gerbils after the ischemic insult resulted in no effect. Moreover, since the daily doses of intraperitoneally administered ginsenoside Rb₁ are as high as 0.7 mg - 1.4 mg determined by the body weight of gerbils (approximately 70 g), judging from the view point of efficacy and effect of ginsenoside Rb₁ administration, intravenous administration of ginsenoside Rb₁ is a superior method for administration than the intraperitoneal administration, and can be easily applied to humans. As well known, an intraperitoneal administration to human can not always be applied except for a partial exception (peritoneal lavage, etc.).

Animals with MCA permanent occlusion (cerebral infarction rats or cerebral embolism rats) used in the present example are obviously more severe than the transient forebrain ischemia model of gerbils and they provide a model close to human disease that is cerebral infarction. Consequently, the fact that the intravenous infusion of ginsenoside Rb₁ starting after cerebrovascular occlusion exhibited a marked favorable effect on rats with permanent MCA occlusion clearly indicates the usefulness, convenience and economical advantage of intravenous infusion of ginsenoside Rb₁ in low doses.

On the other hand, in the previous report by the inventors of the present invention (Sakanaka and Tanaka), in which ginsenoside Rb₁ was directly infused into the cerebral ventricles of animals with MCA permanent occlusion (Zhang B., etal., J. StrokeCerebrovasc. Dis., 7, 1-9, 1998), a significant suppressive effect on cerebral infarction was observed only when the continuous intracerebroventricular infusion of ginsenoside Rb₁ at the dose of 0.6µg/day was conducted after MCA occlusion; and the effect was equal to or a little less than the effect of intravenous administration of ginsenoside Rb₁ as shown in the present example. In the previous report on the intracerebroventricular administration of ginsenoside Rb₁, no therapeutic effect on cerebral infarction was observed when the , other doses of ginsenoside Rb₁ (6µg/day or 0.06µg/day) were continuously infused into the cerebroventricles after MCA permanent occlusion. Consequently, the effective dose range of intracerebroventricularly administered ginsenoside Rb₁ was very narrow and its practical use for clinical medicine was thought to be difficult. Moreover, the actual application of intracerebroventricularly infused ginsenoside Rb₁ to humans appears to be impossible when we consider the balance between its risk and benefit.

Generally, a neuroprotective factor or agent exhibits the maximum effect when directly administered in the cerebral ventricles or into the brain parenchyma, and in case of intravenous or intraperitoneal administration, its effect and efficacy seem to drastically decrease or disappear due to the blood brain barrier that prevents the neuroprotective agent from entering the brain parenchyma, or due to metabolic decomposition of the agent. Consequently, based on the experimental results of intraperitoneal administration or intracerebroventricular administration of ginsenoside Rb₁, the effect and efficacy of intravenously infused ginsenoside Rb₁ could not be anticipated at all.

As clarified by the present invention, however, intravenous administration of ginsenoside Rb₁ reduces effectively the cerebral infarct area of rats with MCA permanent occlusion in a wider dose range than in case of intracerebroventricular administration, and improves learning ability of. the MCA-occluded animals. Ginsenoside Rb₁ is a purified saponin, which is contained in medicinal ginseng, but since it can not be detected in blood after oral administration, a pharmacological action of ginsenoside Rb₁ per se has been substantially denied (Kobashi et al., Medicinal ginseng '95, pp 213-221, Ed. Kumagai A., Kyoritsu Publ.). However, according to the present example, as described in JP98/365560 and PCT/JP99/02550 ("Brain cell or nerve cell-protective agents comprising ginsenoside Rb₁"), it is cleared that intravenous administration of ginsenoside Rb₁ has effect, efficacy and use independent of the medicinal ginseng.

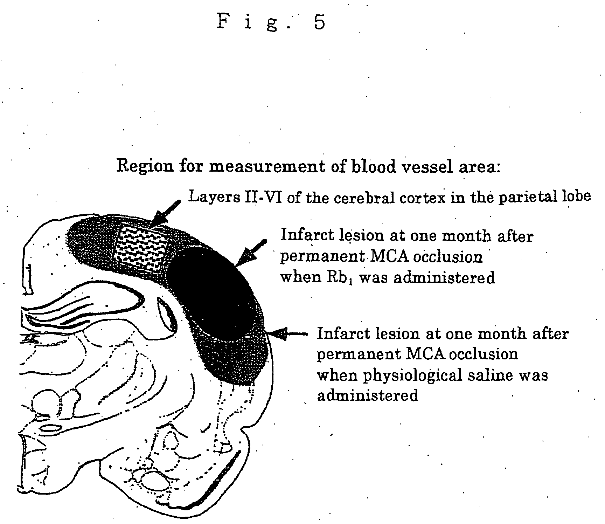

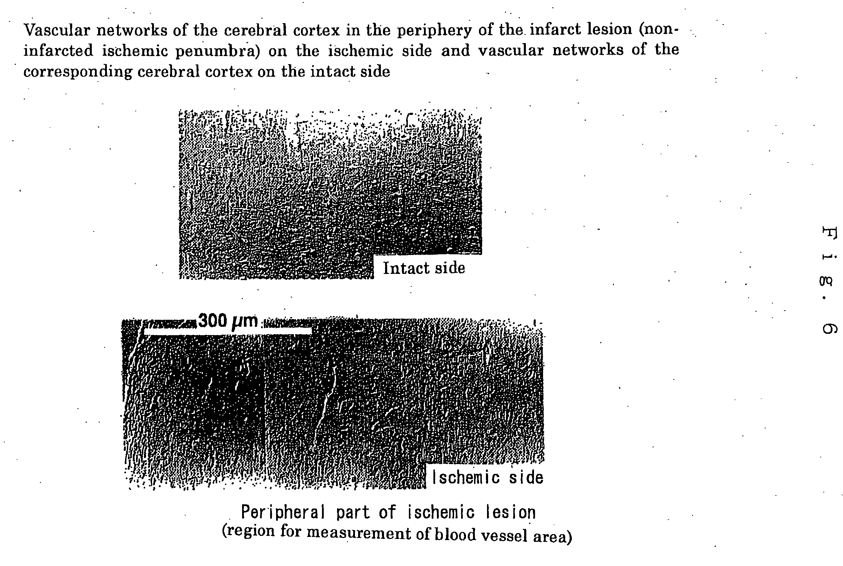

Next, we prepared paraffin sections, 5 µm thickness, obtained from brain samples at the level about 2.8 mm posterior to the bregma. Then, we measured the cerebrovascular area per 1.27 mm² of the non-infarcted ischemic penumbra in the parietal lobe. This was done for each of the cerebral hemisphere of a group to which ginsenoside Rb₁ had been administered intravenously (i.e. the brain tissues had been rescued by administration of ginsenoside Rb₁) (Fig. 5). Four sheets of differential interference contrast micrograph were used for measurement (Fig. 6), and the ratio of the cerebrovascular area was calculated (Table 1).

**Table 1**

| | intact side (%) | ischemic side (%) |
|---|---|---|
| Rb₁: 6µg/day | 7.0 ± 0.64 | 8.0 ± 0.58 |
| Rb₁: 60µg/day | 8.3 ± 0.92 | 9.0 ± 0.52 |

The ratio of the cerebrovascular area was also measured in the control hemispheres (intact side). Table 1 provides a comparison of the ratios of the cerebrovascular areas on the intact side and on the ischemic (lesion) side following the administrations of ginsenoside Rb₁, in doses of 6µg/day and 60 µg/day. Data are represented as a mean ± SE. Statistical analyses were conducted by ANOVA + Fisher's PLSD. As indicated in Table 1, the ratios of the cerebrovascular areas showed no significant difference between the control side and the ischemic side. This indicated that, as a result of intravenous administration of ginsenoside Rb₁ within 28 days after permanent occlusion of the MCA, the cerebrocortical vascular networks in the ischemic penumbra have been almost completely regenerated and reconstructed. In addition, the ratio of the cerebrovascular area in the ischemic penumbra of the parietal lobe is quite naturally, significantly reduced as compared with that on the intact side immediately after permanent occlusion of the MCA.

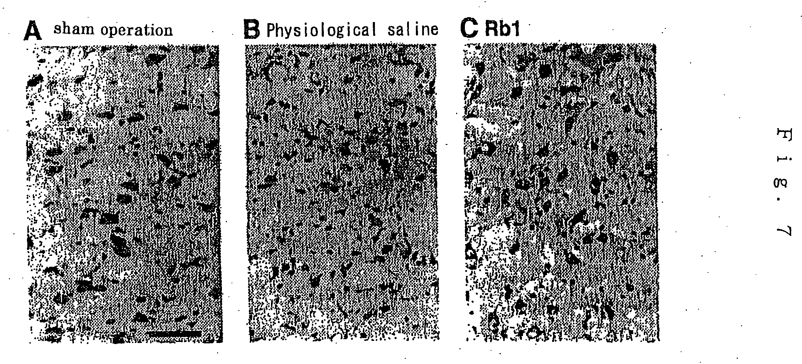

Further, paraffin sections prepared from brain samples at the level 3.6 mm posterior to bregma were subjected to Nissl staining, and the ratio of the left thalamic area to the right thalamic area (ischemic side/intact side × 100) at the same level was measured. The groups administered with ginsenoside Rb₁ showed significantly higher values than the ischemic group administered with vehicle (physiological saline), and they exhibited the value close to that of the sham-operated group. These findings indicate that secondary atrophy of the thalamus generated after cerebrocortical infarction is almost completely inhibited by intravenously administered ginsenoside Rb₁. In addition, we have investigated the histological patterns of the ventral posterior nucleus of the thalamus (VP thalamic nucleus) which has reciprocal synaptic connections (fiber connections) with the ischemic core of the cerebral cortex. Fig. 7A shows the thalamic VP nucleus of a sham-operated animal; Fig. 7B shows the thalamic VP nucleus of an ischemic animal administered with physiological saline; and Fig. 7C shows the thalamic VP nucleus of an ischemic animal administered with ginsenoside Rb₁ (60 µg/day). Comparing the ischemic animal infused with physiological saline (Fig. 7B), with the ischemic animal intravenously infused with ginsenoside Rb₁ (Fig. 7C), reveals that a large number of nerve cells (neurons) survived significantly without the secondary degeneration in the ginsenoside Rb₁-treated VP thalamic nucleus. Consequently, it has been shown that intravenously administered ginsenoside Rb₁ inhibits the secondary degeneration of the thalamus.

We have further investigated the effects of intravenously administered ginsenoside Rb₁ on spinal cord injury, which is an intractable disease causing secondary degeneration of the nervous tissues. When a compression load is added to a spinal cord segment such as the lower thoracic cord, not only nerve cells in the gray matter of that segment but also fiber tracts or pathways in the white matter are damaged. The original damage in the white matter also develops towards the distal region (caudal region) as well as causing secondary degeneration in the efferent neurons or origins of the fiber tracts or pathways damaged, namely the upper nerve cell bodies (i.e. efferent neurons or origins) which project nerve fibers to the fiber tracts or pathways damaged. Accordingly, the damage to the fiber tracts or pathways of the white matter in the lower thoracic cord, which results from the compression load, caused paraplegia of both hindlimbs. Thus, this paraplegia occurs due to the secondary degeneration of the efferent neurons or origins (nerve cell bodies) projecting to the primary lesion of the fiber tracts or pathways in the thoracic cord, and due to secondary degeneration of fiber tracts or pathways distal to the lower thoracic cord (i.e. lumbar and sacral cords). Further, the damage to the lower thoracic cord interrupts the innervation from the upper brain to the lumbar and the sacral cords. As a result, the secondary degeneration of nerve cells or neurons in the gray matter of the lumbar and sacral cords is aggravated to make paraplegia of both hindlimbs irreversible. We used Wistar rats, (weighing about 300 g), which were loaded with 20 g of compression to the lower thoracic cord for 20 minutes, as an animal model for spinal cord injuries.

The rats were anesthetized by inhalation of halothane in a mixture of nitrous oxide and oxygen and were loaded with 20 g of compression to the lower thoracic cord for 20 minutes. More than 30 minutes later, ginsenoside Rb₁ dissolved in physiological saline was infused once into the left femoral vein (12 µg or 60 µg). Subsequently continuous intravenous administration of ginsenoside Rb₁ was performed for 7 days by using an Alza osmotic minipump (12µg/day or 60µg/day). Control animals' and sham-operated animals were administered with the same amount of physiological saline (vehicle). The open field locomotor scores [Basso, Bettie and Bresnakan (BBB) scores] were measured before the loading of spinal cord injury, on the day of spinal cord injury, and from the 1st day to the 7th day after spinal cord injury in order to determine an index of motor functions (Basso D.M. et al., J. Neurotrauma, 13, 343-359, 1996). BBB scores of the sham-operated rats (normal rats) are 20 - 21.

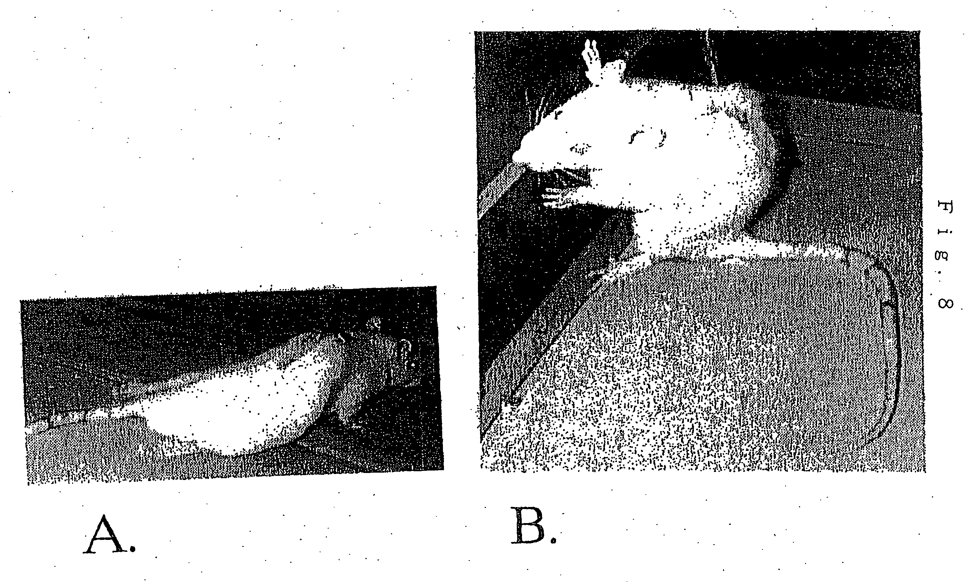

Fig. 8A shows the result of a control rat treated with physiological saline on the 2nd day after spinal cord injury, and Fig. 8B shows the result of a rat administered with ginsenoside Rb₁ (60µg/day) on the 2nd day after spinal cord injury. As shown in Fig. 8A, the rats to which physiological saline was administered and a compression of 20 g was loaded on the lower thoracic cord for 20 minutes exhibited obviously paraplegia in both hindlimbs. However, when ginsenoside Rb₁ (60 µg/day) was intravenously infused after loading a compression of 20 g on the lower thoracic cord for 20 minutes, the paraplegia of both hindlimbs was significantly ameliorated after 2 days, as shown in Fig. 8B. Also the rats treated with ginsenoside Rb₁ could stand up with the aid of a holding bar.

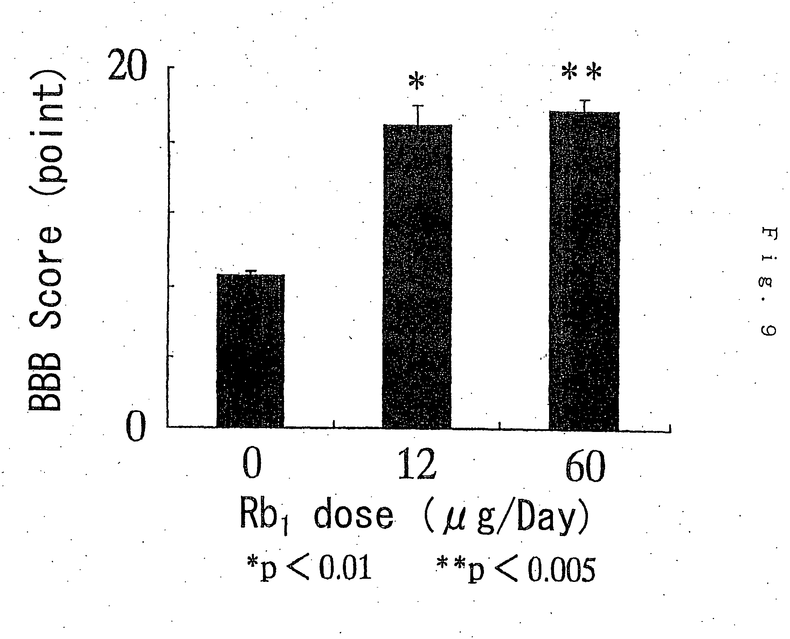

Fig. 9 shows a graph which quantifies the motor ability of rats by using BBB scores on the 7th day after spinal cord injuries. As shown in Fig. 9, the motor ability of rats with spinal cord injuries was significantly ameliorated by the intravenous administration of ginsenoside Rb₁ in a dose-dependent manner. Data are represented as the mean ± SE. Statistical analyses were conducted by the Mann-Whitney U-test.

Solu-Medrol (methylprednisolone), which is used as a remedy for spinal cord injuries in the clinical field at a dose of 30 mg/kg, was intravenously infused into the femoral veins of rats with spinal cord injuries by using the same schedule prepared by the inventors for administering ginsenoside Rb₁. However no significant ameliorating effects on paraplegia were noted. In the Solu-Medrol-administered rats, wound healing 'of the dorsal skin incision was obviously delayed as compared with that of the rats administered with physiological saline. However, in the case of the ginsenoside Rb₁-administered rats, no such adverse effects were noted. This fact indicates that ginsenoside Rb₁ is superior to Solu-Medrol as a remedy for spinal cord injuries and traumatic injuries to the nervous tissues. Furthermore, the required dose of ginsenoside Rb₁ is smaller than that of Solu-Medrol. In addition, unlike Solu-Medrol, ginsenoside Rb₁ has neither an immunosuppressive action nor an ulcer-inducing action. Consequently, ginsenoside Rb₁ is expected to be a quite safe remedy for spinal cord injuries and neuronal traumatic injuries.

Based on the present experimental results using rats with spinal cord injuries, the therapeutic effects on spinal cord injuries of the preparation for intravenous administration comprising ginsenoside Rb₁ are thought to be historically the most potent in the world. Ginsenoside Rb₁ or its metabolites can exhibit extremely potent therapeutic actions to improve the symptoms of spinal cord injuries. This supports the notion that ginsenoside Rb₁ or its metabolites can be a leading compound(s) for the treatment of spinal cord injuries or of neuronal traumatic injuries (neurotrauma).

It is well known that the nervous tissues are more vulnerable to trauma than the other peripheral tissues. The fact that a pharmaceutical composition comprising ginsenoside Rb₁ exhibits significant effects for therapy and treatment of spinal cord injuries suggests that ginsenoside Rb₁ is also effective for the treatment of traumatic injuries to the peripheral tissues.

Since the therapeutic effects of ginsenoside Rb₁ on spinal cord injuries and neuronal traumatic injuries (neurotrauma) are profound, novel pharmaceutical compounds for the treatment of spinal cord injuries can be synthesized by using ginsenoside Rb₁ or its metabolites as a leading compound(s). Further, as a result of identifying the target molecules of ginsenoside Rb₁ or its metabolites, novel compounds which can modify the functions of the target molecules, would be synthesized. Then the development of remedies for spinal cord injuries, neuronal traumatic injuries or traumatic injuries can be directed.

Further, glial cells especially oligodendrocytes are known to enter apoptosis in cases of spinal cord injuries. As a result, demyelination occurs to deteriorate or aggravate the neural symptoms associated with spinal cord injuries (Crowe, M. J. et al., Nature Med. 3, 73-76, 1997; Emery, E. et al., J. Neurosurg. 89, 911-920, 1998). The experimental results in which intravenously administered ginsenoside Rb₁ significantly ameliorates paralysis or paraplegia of both hiridlimbs of rats with spinal cord injuries, indicate that ginsenoside Rb₁ inhibits apoptosis or apoptosis-like cell death of oligodendrocytes and thereby ameliorates the symptoms of spinal cord injuries. Consequently, ginsenoside Rb₁ of the present invention is thought to be useful for prevention, therapy or treatment of brain and nervous diseases accompanied by demyelination (multiple sclerosis, Binswanger's dementia, etc.). Further, the experimental results, in which intravenously administered ginsenoside Rb₁ ameliorates paralysis of both hindlimbs of rats with spinal cord injuries (paraplegia), suggest that injured nerve fibers or nervous tissues can be regenerated as a result of administering ginsenoside Rb₁.

The experimental results described above have demonstrated that the preparations for intravenous administration comprising ginsenoside Rb₁ or its salt can induce regeneration and/or reconstruction of the damaged or reduced cerebrovascular networks in cases of cerebral apoplexy. The preparations have also been shown to exhibit a protective action on brain cells (including glial cells) and a protective action on nerve cells (JP98/365560 and PCT/JP99/02550: "Brain cell or nerve cell-protective agents comprising ginsenoside Rb₁"). As a result, the preparations protect brain tissues. It has also been demonstrated that the preparations for intravenous administration comprising ginsenoside Rb₁ or its salt inhibit not only the primary lesions in the nervous tissues, but also the secondary lesions in brain regions which have synaptic connections (fiber connections) with the primary lesions. Further, the pharmaceutical composition comprising ginsenoside Rb₁ is expected to be an epoch-making remedy for spinal cord injuries and neural traumatic injuries (neurotrauma), as well as exhibiting efficacy and effectiveness against traumatic injuries to the peripheral tissues.

Ginsenoside Rb₁, or its salt, is known to be a component of medicinal ginseng and is very low in toxicity.

### Examples

The present invention will be explained in detail by concrete examples, but the present invention is not limited within these examples.

### Example 1 (Experiment on intravenous infusion of ginsenoside Rb₁)

Male SH-SP rats, at the age of 12-13 weeks, (stroke-prone spontaneously hypertensive rat: weighing 250 - 300 g), were used. Animals were housed in an air-conditioned room furnished with a 12:12 hour light-dark cycle, and water and feeds were supplied ad libitum. The mean blood pressure of the animals was 203±6.9 mmHg. The following experiments were conducted in accordance with the Guide for Animal Experimentation at Ehime University School of Medicine. The cortical branch of the left middle cerebral artery (MCA) of SH-SP rats were coagulated and cut, while their rectal temperature was maintained at 37±0.2°C under inhalation anesthesia.

Immediately after MCA permanent occlusion, 6.0µl of physiological saline containing ginsenoside Rb₁ at a concentration of 1µg/µl or 0.1µg/µl (60µg or 6µg as ginsenoside Rb₁) was injected once into the left femoral vein. Then a catheter connected to the Alza osmotic minipump implanted subcutaneously in the back of each animal was inserted into the same vein from the point of the single injection of ginsenoside Rb₁. Physiological saline containing ginsenoside Rb₁ was filled in advance in the said osmotic minipump, and ginsenoside Rb₁ in a dose of 60µg/day or 6µg/day was continuously infused into the blood stream through the catheter placed in the left femoral vein for 28 days. Flow rate of the ginsenoside Rb₁-containing solution was 0.25µl/hour. Control animals with MCA permanent occlusion (ischemic control animals) and sham-operated animals received the same amount of physiological saline.

### Example 2 (Water maze tests)

After the MCA permanent occlusion according to the method of the inventors of the present invention (Sakanaka and Tanaka) (Igase, K. et al., J. Cereb. Blood Flow Metab., 19, 298-306, 1999; Zhang B. et al., J. Stroke Cerebrovasc. Dis., 7, 1-9, 1998), water maze tests were performed for 4 days at the 2nd week and the 4th week, respectively, and place navigation abilities of the SH-SP rats were determined.

Results are shown in Fig. 1. In Fig. 1, the left drawing shows the results of the 2nd week and the right drawing shows the results of the 4th week after permanent MCA occlusion. In Fig. 1, closed circles (●) indicate the results of rats with sham-operation; and open circles (○) indicate the results of MCA-occluded rats administered with only physiological saline; closed squares (■) indicate the results of MCA-occluded rats administered with ginsenoside Rb₁ in a dose of 6µg/day and open squares (□) indicate the results of MCA-occluded rats administered with ginsenoside Rb₁ in a dose of 60µg/day. Data are represented as a mean±SE. Statistical analyses were conducted by ANOVA + Fisher's PLSD.

No significant differences in swimming speed were detected among the four experimental groups.

### Example 3 (Measurement of ratio of lesion)

After termination of the water maze tests at the 4th week, the SH-SP rats were anesthetized with chloral hydrate, and they were perfused and fixed transcardially with 0.1 mole phosphate buffer containing 4% paraformaldehyde. The brains were dissected out and cerebrocortical infarcted areas were photographed. Areas of the left cerebral hemispheres and the left cerebrocortical infarct lesions were measured on the photographs by using an image analysis device. The left cerebrocortical infarcted areas were divided by the left cerebral hemispheric areas to calculate ratios of the cerebrocortical infarction (%). Results are shown in Fig. 2. Data are represented as mean ± SE. Statistical analyses were performed by Mann-Whitney U-test.

An actual case of a cerebral infarct lesion of the group administered with physiological saline and an actual case of a cerebral infarct lesion of the ginsenoside Rb₁ (6µg/day)-administered group are shown in Fig. 3A and Fig. 3B, respectively.

Fig. 4 is a schematic drawing summarizing the results of the present experiments. In rats administered with physiological saline, the size of cerebral infarction remained large, and in the water maze tests, it took a long time for the rats to escape onto the goal platform. Contrary, in rats administered with ginsenoside Rb₁ of the present invention, the infarct area was reduced, and as a result, in the water maze tests, only short time was required for the rats to arrive at the goal platform.

The isolated brain was embedded in paraffin, and paraffin sections, 5µm thick, at the level approximately 2.8 mm posterior to bregma including the cerebrocortical infarct lesion were prepared. Then, we measured the cerebrovascular area per 1.27 mm² of the ischemic penumbra rescued by intravenous administration of ginsenoside Rb₁. This was done for each of the cerebral hemispheres of the groups to which ginsenoside Rb₁ had been administered intravenously (i.e. the brain tissues had been rescued by administering ginsenoside Rb₁) (Fig. 5). Four sheets of differential interference contrast micrograph were used for measurement (Fig. 6), and the ratio of the cerebrovascular area was calculated (Table 1). The ratio of cerebrovascular area was also measured in the control hemisphere (intact side). As indicated in Table 1, the ratio of cerebrovascular area showed no significant difference between the intact side and the ischemic side.

Fig. 5 is a drawing showing the region for measurement of blood vessel area in the non-infarcted ischemic penumbra of the parietal lobe in a 5µm thick section of the brain. In Fig. 5, the part enclosed with a rectangle is the region for measurement of blood vessel area: cerebrocortical layers II - IV of the parietal lobe; the part painted with black in Fig. 5 is the infarct lesion when ginsenoside Rb₁ was administered; and the part shown in gray in Fig. 5 is the infarcted lesion when physiological saline was administered.

Fig. 6 consists of photographs instead of drawing, which show vascular networks in the non-infarcted ischemic penumbra of the cerebral cortex on the ischemic side, and vascular networks in the corresponding cerebral cortex on the intact side. The upper part shows the intact side and the lower part the ischemic side.

Table 1 shows the ratio of blood vessel area in the non-infarcted ischemic penumbra of the parietal lobe in the brain sections 5µm thick. Data are represented by mean ± SE. Statistical analyses are conducted by ANOVA + Fisher's PLSD. N = 5.

Further, paraffin sections prepared from brain samples at the level 3.6 mm posterior to bregma were subjected to Nissl staining, and the ratio of the left thalamic area to the right thalamic area (ischemic side/intact side × 100) at the same level was measured. The groups administered with ginsenoside Rb₁ showed significantly higher values than the ischemic group administered with vehicle (physiological saline), and they exhibited the values close to that of the sham-operated group.

Results are shown in Table 2.

**Table 2**

| | n | ratio of area (%) | count of nerve cells |
|---|---|---|---|
| physiological saline | 8 | 86.4 ± 8.1 | 10.1 ± 5.5 |
| Rb₁: 6µg/day | 5 | 95.9 ± 3.3* | 30.6 ± 2.9** |
| Rb₁: 60µg/day | 8 | 95.3 ± 2.4* | 31.5 ± 3.6** |
| Sham-operated group | 8 | 98.8 ± 5.3 | 58.5 ± 4.7 |

Table 2 shows the ratio of the left thalamic area to the right thalamic area and numbers of nerve cells (neurons) per 0.099 mm² of the ventral posterior nucleus of the thalamus. Data are represented as mean ± SE. Statistical analyses were conducted by Mann-Whitney U-test. In Table 2, *: p < 0.05, **: p < 0.01. Further, in the ventral posterior nucleus of the thalamus (VP thalamic nucleus) having close synaptic connections (fiber connections) with the ischemic core, large numbers of nerve cells significantly survived without suffering from the secondary degeneration in the ischemic groups administered intravenously with ginsenoside Rb₁ as compared with the ischemic group intravenously infused with vehicle (physiological saline).

### Example 4 (Effect of intravenous infusion of ginsenoside Rb₁ on rats with spinal cord injuries)

The rats were anesthetized with inhalation of halothane in a mixture of nitrous oxide and oxygen and were loaded with 20 g of compression to the lower thoracic cord for 20 minutes. More than 30 minutes later, ginsenoside Rb₁ dissolved in physiological saline was injected once into the left femoral vein (12µg or 60µg), thereafter continuous intravenous administration of ginsenoside Rb₁ was performed for 7 days by using an Alza osmotic minipump (12µg/day or 60µg/day). Control animals and sham-operated animals were administered with the same amount of physiological saline (vehicle). The open field locomotor scores [Basso, Bettie and Bresnakan (BBB) scores] were measured before loading spinal cord injury, on the day of spinal cord injury and from the 1st day to the 7th day after the spinal cord injury for use as an index of motor functions (Basso D.M. et al., J. Neurotrauma, 13, 343-359, 1996).

Fig. 8A shows the result of a control rat administered with physiological saline on the 2nd day after spinal cord injury, and Fig. 8B shows the result of a rat administered with ginsenoside Rb₁ (60/µg/day) on the 2nd day after spinal cord injury. As shown in Fig. 8A, the rat, to which physiological saline was administered and the compression of 20 g was loaded on the lower thoracic cord for 20 minutes, obviously exhibited paraplegia in both hindlimbs. However, when ginsenoside Rb₁ (60 µg/day) was intravenously infused after loading the compression of 20 g on the lower thoracic cord for 20 minutes, the paraplegia of both hindlimbs was significantly ameliorated after 2 days, as shown in Fig. 8B, and the rat could stand up with the aid of a holding bar.

Fig. 9 shows a graph which quantifies the motor ability of rats by using BBB scores at the 7th day after spinal cord injuries. As shown in Fig. 9, the motor ability of rats with spinal cord injuries was significantly ameliorated by intravenous administration of ginsenoside Rb₁ in a dose-dependent manner. Data are represented as mean ± SE. Statistical analyses were performed by Mann-Whitney U-test. *: p < 0.01, **: p < 0.005.

### Example 5 (Prevention, therapy or treatment of bedsore by ginsenoside Rb₁)

Decubitus of bedridden patients and aged subjects aggravates the systemic condition, and markedly deteriorates QOL (Quality of Life). At early stages of decubitus, skin of lesion develops rubefaction or rubescence. The fact that almost no preparations for external use show effect and efficacy on the local region of cutaneous lesion by applying externally, is the large problem in the field of dermatology.

Ginsenoside Rb₁ is mixed with water-soluble bases or fat-soluble bases with or without glucose to make the external use preparations for skin (cream or ointment). The preparations are applied topically on the lesion of decubitus and its surrounding region (penumbra) until the decubital lesion is cured, reduced or unchanged. In that occasion, the amount of ginsenoside Rb₁ in the base is adjusted so that the extracellular concentrations of ginsenoside Rb₁ in the lesion are kept at 1 ng/ml or less, preferably 10 pg/ml or less, more preferably 100 fg/ml or less. If sufficient effect can not be obtained by the topical application of the external use preparation for skin containing ginsenoside Rb₁, intravenous administration of ginsenoside Rb₁ is combined.

### Example 6 (Prevention, therapy or treatment of corneal injury using ginsenoside Rb₁)

It is well known that corneal injuries occur at the application of contact lens or after corrective operation for myopia using an excimer laser, however almost no eye drops, which can protect keratic tissues, are known at present.

Eye drops are prepared by mixing ginsenoside Rb₁ with any one of basal ophthalmic solutions. The eye drops are applied for necessary times continuously to the patients with the keratic or corneal injuries, and are continuously applied until ameliorating or healing the keratic lesion. In that occasion, the amount of ginsenoside Rb₁ in the basal solution is adjusted so that the extracellular concentrations of ginsenoside Rb₁ in the keratic lesion tissues are kept at 1 ng/ml or less, preferably 10 pg/ml or less, more preferably 100 fg/ml or less.

### Example 7 (Protection of cornea for transplantation by ginsenoside Rb₁)

The keratoplasty is frequently carried out in the field of ophthalmology as the method of treatment with the highest probability of success in the transplantation medicine. However, since cells in the corneal tissues for transplantation are certainly going to death during the term after dissecting out the tissues from the donor to perform keratoplasty, such term is'the rate-limiting factor for the successful corneal graft. After collecting the cornea for transplantation, ginsenoside Rb₁ is admixed to the conventional corneal preservative solution at 1 ng/ml or less, preferably 10 pg/ml or less, and more preferably 100 fg/ml or less to protect cornea for transplantation.

### Example 8 (Prevention, therapy or treatment of chorea by ginsenoside Rb₁)

Among neurodegenerative diseases, chorea (Huntington's chorea) is a representative single genetic disease and CAG repeats coding polyglutamine appear to be the etiology of chorea, but no method for therapy is developed. Transfection of the causal gene of chorea, mutant huntingtin, into cultured neural cells originating in the striatum causes the cells to fall into apoptosis-like neuron death. However, if Bcl-X_{L} is over-expressed in causes the cultured nerve cells together with the mutant huntingtin, the cell death is almost completely suppressed (Saudou, F., et al., Cell, 95, 55-66, 1988). Consequently, as we have found out in JP98/365560 and PCT/JP99/02550 ("Brain cell or nerve cell-protective agents comprising ginsenoside Rb₁"), if ginsenoside Rb₁, which upregulates Bcl-X_{L} protein expression in the brain tissues, is nasally or intravenously administered before or after the onset of chorea, the efficacy and effect is highly expected. For the treatment of polyglutamine diseases other than chorea (Machedo-Joseph disease, detatorubral-pallidoluysian atrophy, etc.), nasal or intravenous administration of ginsenoside Rb₁ is likely to be effective. Further, as found out in the present invention, ginsenoside Rb₁ is expected to inhibit development of the secondary degeneration from the primary lesion (striatal lesion) of chorea to the other brain regions, which have fiber connections with the striatum.

To the humans, who have determined to have chorea or the other polyglutamine diseases in future by gene diagnosis, (estimated body weight 60 kg), or to patients, who have already developed chorea or the other polyglutamine diseases (estimated body weight 60 kg), a preferable amount of ginsenoside Rb₁ is administered nasally or intravenously until the pathologic conditions are ameliorated or stabilized. The amount of intravenous administration of ginsenoside Rb₁ for the treatment of polyglutamine diseases is equivalent to the amount required for the treatment of acute cerebral apoplexy. Dosage of nasal administration can be adjusted to maintain blood levels equal to the intravenous administration of ginsenoside Rb₁.

### Example 9 (Prevention, therapy or treatment of dilated cardiomyopathy by ginsenoside Rb₁)

Dilated cardiomyopathy is a disease which shows reduced heart function and cardiac dilation as a result of noncausal myocardial cell death (myocardial cell degeneration). Reduced heart function is deteriorated progressively and develops cardiac failure to death. When cardiac failure is developed, it has been thought to be no other therapy except for heart transplantation. Perhaps, when myocardial cells of patients with dilated cardiomyopathy is going to death, the cytoprotective gene product Bcl-X_{L} protein, which is contained abundantly in the myocardial cells, is suspected to decrease. Consequently, the decrease in Bcl-X_{L} protein is terminated by intravenous or nasal administration of ginsenoside Rb₁ (JP98/365560 and PCT/JP99/02550: "Brain cell or nerve cell-protective agents comprising ginsenoside Rb₁") and myocardial cell death of the patients can be inhibited, and heart function of the patients may be maintained for a long time.

For patients (estimated body weight 60 kg), who are diagnosed as dilated cardiomyopathy, a proper amount of ginsenoside Rb₁ is promptly administered nasally or intravenously until pathologic condition of the patients is ameliorated or ceases from deteriorating. Ginsenoside Rb₁ and the other drugs for ingestion, for treatment of cardiomyopathy and cardiac failure, such as β-blockers, calcium antagonists, ACE-inhibitors, diuretics can be administered in combination. When ginsenoside Rb₁ is administered to patients with diseases of peripheral organs such as cardiac diseases, amount of ginsenoside Rb₁ equal to that for patients with central nervous system (CNS) diseases or amount of 1/10 - 1/100,000 thereof can be preferably selected.

### Industrial Applicability

The present invention provides the efficacious promoters of cerebrovascular regeneration and/or reconstruction comprising preparations for intravenous administration of low concentrations of ginsenoside Rb₁, which can be used after cerebral apoplexy (including cerebral hemorrhage, subarachnoidal hemorrhage, cerebral infarction, cerebral thrombosis, cerebral embolism, transient cerebral ischemic attack). Namely, the present invention relevant to ginsenoside Rb₁ provides drugs or pharmaceutical compositions which can be administered to patients, who are suspected to have cerebral apoplexy, in ambulance cars. Since in the actual cases of acute cerebral apoplexy the pathological condition is prevalently aggravated within 2 weeks after onset, sufficient effect can be expected if ginsenoside Rb₁ of the present invention can be administered during this term.

Further, since the pharmaceutical compositions of the present invention exhibit novel effect and efficacy for vascular regeneration and/or reconstruction, they appear to be effective for diseases with the major symptom of blood flow disorder (aortitis syndrome, collagen diseases, acute peripheral arterial occlusive diseases, thromboangitis obliterans, arteriosclerosis obliterans, thrombophlebitis, diabetic retinopathy, diabetic nephropathy, retinal embolism, Raynaud's disease, Raynaud's syndrome, myocardial infarction, decubitus, peripheral circulatory failure, angina pectoris, ischemia-reperfusion injuries of liver, kidney or heart, etc.). As described in JP98/365560 and PCT/JP99/02550: "Brain cell or nerve cell-protective agents comprising ginsenoside Rb₁", in the diseases with blood flow disorders, to suppress cell death in the tissues suffering from blood flow disorder is the efficacy of ginsenoside Rb₁, which should not be overlooked. Consequently, in the diseases of either central or peripheral tissue origin, in which blood flow disorder is the major symptom ginsenoside Rb₁ can reduce the damages or injuries of tissues or cells suffering from blood flow disorders through at least two action mechanisms.

Generally, the etiology of the primary lesion of nervous or brain diseases varies from disease to disease. For example, cerebral apoplexy is caused by cerebrovascular, obstruction or blood flow failure. Neurodegenerative diseases are developed as the results' of a complex mixture of gene abnormality, environmental factors and life-style habits, and thus it is not always easy to prevent progress of the primary lesion of each neurodegenerative disease by determining its primary cause(s) and excluding the cause(s). On the other hand, whatever the causes of the primary lesion in cerebral apoplexy or in neurodegenerative diseases are, once the primary lesion is formed, various brain regions having synaptic connections (fiber connections) with the primary lesion site develop the secondary degeneration. Perhaps, in the secondary degeneration of nervous tissues associated with such synaptic connections (fiber connections), at least in part certain common mechanism(s) may be involved. In the present invention, intravenous administration of low concentrations of ginsenoside Rb₁ can effectively suppress the secondary degeneration of the thalamus after cerebrocortical infarction, and the secondary degeneration of the nervous tissues after spinal cord injuries. Based on these facts, intravenous administration or nasal administration of ginsenoside Rb₁ is effective for suppression of the secondary degeneration of the nervous tissues subsequent to the other types of cerebral apoplectic primary lesions (cerebral hemorrhage, subarachnoidal hemorrhage or transient cerebral ischemic attack) and subsequent to the primary lesions of the other brain or nervous diseases (Alzheimer's disease, Pick's disease, spinocerebellar degeneration, Parkinson's disease, demyelinating diseases, chorea, polyglutamine diseases, cerebral palsy, amyotrophic lateral sclerosis, glaucoma, senile macular degeneration, AIDS encephalopathy, hepatic encephalopathy, encephalitis, progressive supranuclear palsy, multiple sclerosis, diabetic retinopathy, diabetic neuropathy, retinal detachment, retinal pigment degeneration, carbon monoxide poisoning, newborn infant asphyxia, peripheral nerve injuries, spastic paraplegia, progressive supranuclear palsy, vascular lesions in the spinal cord, mitochondrial encephalopathy, meningitis, etc.). Further, administration of ginsenoside Rb₁ is effective for compression or paralysis of the spinal cord and/or its roots accompanied with herniaton of intervertebral disk, spinal canal stenosis, spondylolysis, slipping diseases, cervical spondylolysis and ossification of the posterior longitudinal ligament. It is also effective for facial nerve paralysis. These efficacy and effects of ginsenoside Rb₁ can delay aggravation of the pathological conditions of patients suffering from intractable brain or nervous diseases and can contribute to the improvement of QOL (Quality of Life).

Ginsenoside Rb₁ can be expected to protect hepatic cells (hepatocytes) and renal cells in cases of acute and chronic hepatitis or nephritis. In addition, it may ameliorate prognosis of patients with hepatitis and nephritis by facilitating the regeneration and/or reconstruction of vascular networks once destroyed by hepatitis and nephritis. Further, ginsenoside Rb₁ exhibits effect and efficacy on healing of decubitus or wound through its cytoprotective action and vascular regeneration and reconstruction-promoting actions. In that case, ginsenoside Rb₁ can be used not only as an agent for intravenous administration but also as an agent for external use and local injection to lesion. Further, method for administration of ginsenoside Rb₁ can be selected among various routes of administration such as subcutaneous injection, intramuscular injection, eye drops, nasal application, inhalation, suppository, oral administration, sublingual administration, percutaneous administration, etc. In case of oral administration of ginsenoside Rb₁, sole administration of ginsenoside Rb₁ can not be expected to exhibit good effects, and thus it is necessary to make oral administration of ginsenoside Rb₁ by combining, encapsulating or binding with carriers which inhibit decomposition in the digestive tract or promote absorption in the digestive tract. If metabolites of ginsenoside Rb₁ showing equivalent or more effect or efficacy as compared with ginsenoside Rb₁ are identified, such metabolites can be administered by the conventional methods to patients with the diseases described above, to which application of ginsenoside Rb₁ can be expected to be effective. After preparing dispersing agents with ginsenoside Rb₁ of the present invention and any polymer compounds, the mixture is spray dried to apply to patients via any routes of administration. Any routes of administration can be selected after coating ginsenoside Rb₁ with microgranules of polymer.

Ginsenoside Rb₁ is thought to be effective for protection and/or maintenance of cultured keratinocyte sheets for skin graft. Injuries of cells and destruction of vascular networks of organs for transplantation (liver, kidney, heart, spleen, lung, digestive tract, cornea, blood vessel, etc) can be inhibited by immersing or perfusing such organs with solutions containing low concentrations of ginsenoside Rb₁ before performing transplantation operation, and thereby results of transplantation can be improved (in JP98/365560 and PCT/JP99/02550: "Brain cell or nerve cell-protective agents comprising ginsenoside Rb₁"). Further, ginsenoside Rb₁ is thought to be effective for protection and/or maintenance of blood cell components and platelets and for protection and/or maintenance of frozen ovum or sperm.

Intravenous administration of ginsenoside Rb₁ of the present invention ameliorates significantly paraplegia of animals with spinal cord injuries. It is well known that the nervous tissues are more vulnerable to trauma than the other peripheral tissues. The fact that the pharmaceutical composition comprising ginsenoside Rb₁ exhibits significant' effects for therapy and treatment of spinal cord injuries suggests that ginsenoside Rb₁ may also be effective for treatment of traumatic injuries to the peripheral tissues as well as the central nervous tissues. As shown in example 4, rats with spinal cord injuries, to which compression is loaded on the lower thoracic cord, can stand up as a result of intravenous administration of ginsenoside Rb₁ after the injuries. The physiological saline (i.e. vehicle)-infused rats with spinal cord injuries can not stand up as well as exhibiting paraplegia of both hindlimbs. On the basis of these facts, the therapeutic effects of ginsenoside Rb₁ for'spinal cord injuries are thought to be the most potent as far as we know. Consequently, it is expected that, in the future, ginsenoside Rb₁ or its metabolites will be used as a leading compound(s) in development of various remedies for spinal cord injuries and traumatic injuries to the nervous tissue. Further, as a result of identifying the target molecule(s) of ginsenoside Rb₁, novel compounds which can modify the functions of the target molecules, can be synthesized. As such, the present invention is essential for developing remedies for spinal cord injuries and neuronal traumatic injuries (neurotrauma) which have threatened humans in history.

Once a major blood vessel of brain such as MCA is permanently occluded, even if nerve cells in the ischemic penumbra are rescued initially by the action of a powerful neuroprotectant, and if cerebrovascular regeneration and/or reconstruction can not be achieved, meanwhile nerve cells in the ischemic penumbra once rescued are possibly going to death. As a result, expansion of cerebral infarct lesion occurs frequently within one month after MCA permanent occlusion. Such a time-dependent expansion of cerebral infarct lesion is frequently observed in the clinical field, and this is the pathological ground for the worst prognosis of patients with cerebral infarction. Quite unfortunately, there have been no drugs which could reduce volume of lesion to about 1/4 compared to that of non-administered group even in acute phase as well as one month after the onset of cerebral infarction by intravenous administration after MCA permanent occlusion. In the present invention, the intravenous administration of ginsenoside Rb₁ after the onset of cerebral infarction can reduce the lesion to about 1/4 compared to the non-administered group at one month after permanent MCA occlusion. Based on this fact, ginsenoside Rb₁ appears to be the most effective nerve cell protector in the human history. Consequently, various nerve cell or brain cell protectors can be newly developed by using ginsenoside Rb₁ or its metabolites as a leading compound(s). In addition, as the results of modifying a part(s)of the chemical structure of ginsenoside Rb₁ to prepare prodrugs thereof, any routes of administration and any methods for administration can be selected as described hereinfefore. Finally, the pharmaceutical compositions of the present invention exhibit almost no adverse effects, consequently the present invention provides highly safe pharmaceuticals.

## Claims

1. A pharmaceutical composition comprising ginsenoside Rb₁, its metabolites or salt thereof for prevention, treatment or therapy of diseases caused by injuries to the nervous tissues or to the spinal cord.

2. The pharmaceutical composition for prevention, treatment or therapy according to claim 1, comprising suppressing the secondary degeneration of the nervous tissues caused by injuries to the nervous tissues.

3. The pharmaceutical composition for prevention, treatment or therapy according to claim 2 wherein the nervous tissue having the secondary degeneration is the thalamus after cerebrocortical infarction.

4. The pharmaceutical composition for prevention, treatment or therapy according to claim 2 or 3 wherein disease causing the secondary degeneration of the nervous tissues is spinal cord injury.

5. The pharmaceutical composition for prevention, treatment or therapy according to claim 1 wherein said prevention, treatment or therapy is achieved by means of vascular regeneration and/or reconstruction of the injured nervous tissues or spinal cord.

6. The pharmaceutical composition for prevention, treatment or therapy according to claim 5 wherein the blood vessels are cerebral blood vessels.

7. The pharmaceutical composition for prevention, treatment or therapy according to claim 1 wherein the diseases caused by injuries to the nervous tissues or to the spinal cord are traumatic injuries.

8. The pharmaceutical composition for prevention, treatment or therapy according to claim 1 wherein the diseases caused by injuries to the nervous tissues or to the spinal cord are demyelination.

9. The pharmaceutical composition for prevention, treatment or therapy according to claim 1 wherein the diseases caused by injuries to the nervous tissues or to the spinal cord are spinal cord injuries.

10. The pharmaceutical composition for prevention, treatment or therapy according to claim 8 or 9 comprising suppressing apoptosis or apoptosis-like cell death of oligodendrocytes.

11. The pharmaceutical composition for prevention, treatment or therapy according to any one of claims 1 - 10 comprising the preparations for intravenous administration.

12. The pharmaceutical composition for prevention, treatment or therapy according to any one of claims 1 - 10 comprising the preparations for a single intravenous infusion or the preparations for continuous intravenous administration.

13. A pharmaceutical composition comprising ginsenoside Rb₁, its metabolites or salt thereof for promoting vascular regeneration and/or reconstruction.

14. A pharmaceutical composition comprising ginsenoside Rb₁, its metabolites or salt thereof for prevention, treatment or therapy of the secondary degeneration of the nervous tissues.

15. A pharmaceutical composition comprising ginsenoside Rb₁, its metabolites or salt thereof for prevention of deterioration of traumatic injuries to the nervous tissues or to the spinal cord or for treatment or therapy of traumatic injuries to the nervous tissues or to the spinal cord.

16. The pharmaceutical composition according'to claim 15 wherein traumatic injuries are spinal cord injuries, neurotrauma, or head injuries.

17. A pharmaceutical composition comprising ginsenoside Rb₁, its metabolites or salt thereof for suppressing apoptosis or apoptosis-like cell death of oligodendrocytes.

18. The pharmaceutical composition according to claim 17 wherein diseases causing apoptosis or apoptosis-like cell death of the oligodendrocytes are spinal cord injuries.

19. A pharmaceutical composition comprising ginsenoside Rb₁, its metabolites or salt thereof for prevention, treatment or therapy of demyelination.

20. The pharmaceutical composition according to claim 19 wherein diseases causing demyelination are spinal cord injuries.

21. A method for exploring novel active compounds or compositions for prevention, treatment or therapy of diseases of the nervous tissues or the spinal cord comprising using ginsenoside Rb₁ or its metabolites as a leading compound(s).

22. The method according to claim 21 wherein the diseases of the nervous tissues or the spinal cord are the diseases caused by injuries to the nervous tissues or to the spinal cord.

23. The method according to claim 21 or 22 wherein the diseases of the nervous tissues or the spinal cord are spinal cord injuries or neurotrauma.

24. Pharmaceutical compositions for prevention, treatment or therapy of diseases of the nervous tissues or the spinal cord as obtained by the method according to any one of claims 21 - 23.

25. Use of ginsenoside Rb₁ or its metabolites as the leading compound(s) for exploring novel active compounds or compositions for prevention, treatment or therapy of diseases of the nervous tissues or the spinal cord.

26. Use of ginsenoside Rb₁ or its metabolites as the leading compound(s) for exploring novel brain cell-protective agents or nerve cell-protective agents.

27. Use of ginsenoside Rb₁, its metabolites or salt thereof for production of pharmaceutical compositions for prevention, treatment or therapy of diseases caused by injuries to the nervous tissues or to the spinal cord.
